# EUROPÄISCHE PATENTSCHRIFT

(11) **EP 1 354 594 B1**
(45) Veröffentlichungstag und Bekanntmachung des Hinweises auf die Patenterteilung: **16.04.2008**
(21) Anmeldenummer: 02008624.5
(22) Anmeldetag: 17.04.2002
(51) Int. Cl.: A61K 36/45, A61K 31/05, A61K 31/015, A61P 27/02

(54) **Wirkstoffmischungen von Extrakten der Gattung Vaccinium und Carotinverbindungen**
Combinations of an extract of a vaccinium type plant and a carotene
Combinaison d'un extrait de plante du genre vaccinium avec un carotène

(43) Veröffentlichungstag der Anmeldung: 22.10.2003
(73) Patentinhaber: Cognis IP Management GmbH, 40589 Düsseldorf (DE)
(72) Erfinder: Krächter, Hans-Udo, Dr., 14482 Postdam (DE); Mehling, Annette, Dr., 42349 Wuppertal (DE); Arias, Carmen, 08190 Sant Cugat del Vallés (ES); Fabry, Bernd, Dr., 41352 Korschenbroich (DE)

(56) Entgegenhaltungen:
- WO-A-01/85183
- WO-A-01/91765
- US-A- 5 955 102
- US-A- 6 103 756

## Beschreibung

### Gebiet der Erfindung

Die Erfindung befindet sich auf dem Gebiet der Nahrungsmittelzusatzstoffe und botanischen Extrakte und betrifft neue Mikrokapseln mit einem Gehalt an Wirkstoffzubereitungen zur oralen Aufnahme, ein Verfahren zu deren Herstellung sowie deren Verwendung..

### Stand der Technik

Aus der Geschichte des 2. Weltkrieges ist bekannt, dass auf beiden Seiten der kriegsführenden Parteien umfangreiche Versuche zur Steigerung der Leistungsvermögen der Soldaten durch Verabreichung von Wirkstoffen durchgeführt wurden. Die Bandbreite der damit beabsichtigten Wirkungen reichte von der Enthemmung bis zum Berserkertum und dem völligen Verlust des Angst- und Schmerzgefühles. Abgesehen davon, dass solche Versuche aus ethischer Sicht höchst zweifelhaft und daher abzulehnen sind, waren die Ergebnisse auch wenig erfolgreich. Zu den wenigen dokumentierten und aus ethischer Sicht kaum zu beanstandenden Untersuchungen gehört die Verabreichung von Heidelbeersaft bzw. Heidelbeerextrakten zur Verbesserung des Nachtsichtvermögens von Kampfpiloten, die insbesondere in der britischen Royal Airforce verbreitet war. Rückschauend kann der damals empirische Befund wohl durch Stimulation des Sehnervs durch die in den Heidelbeeren vorkommenden Anthocyanoside erklärt werden. Letztlich hat sich die Verwendung von derartigen Zubereitungen zur Verbesserung des Sehvermögens jedoch nicht durchsetzen können, da der Effekt in der Praxis entweder nicht ausgeprägt und/oder nicht ausreichend reproduzierbar, d.h. stark vom Probanden abhängig war.

Die Aufgabe der vorliegenden Erfindung hat daher darin bestanden, die bekannte Wirkung des Heidelbeerextraktes bzw. der darin enthaltenen wesentlichen Wirkstoffe durch Identifizierung eines geeigneten Synergisten so zu steigern, dass damit die Herstellung eines verbesserten Medikamentes zur Verbesserung des menschlichen Sehvermögens, speziell des Nachtsichtvermögens, möglich wird. Eine weitere Aufgabe hat darin bestanden, solche Wirkstoffmischungen in einer Darreichungsform zur Verfügung zu stellen, welche ihre verzögerte Freisetzung nach oraler Aufnahme bei höchst möglicher toxikologischer Unbedenklichkeit erlaubt.

### Beschreibung der Erfindung

Gegenstand der Erfindung sind Mikrokapseln mit mittleren Durchmessern im Bereich von 0,1 bis 5 mm, bestehend aus einer Hüllmembran und einer die Wirkstoffe enthaltenden Matrix, die dadurch erhältlich sind, dass man
(a) aus Gelbildnern, Chitosanen und einer Wirkstoffmischung enthaltend (i) Extrakte der Gattung Vaccinium bzw. Anthocyanoside und (ii) Carotinverbindungen eine Matrix zubereitet, und diese mit wässrigen Lösungen anionischer Polymere behandelt;
   oder
(b) aus Gelbildnern, anionischen Polymeren und einer Wirkstoffmischung enthaltend (i) Extrakte der Gattung Vaccinium bzw. Anthocyanoside und (ii) Carotinverbindungen eine Matrix zubereitet und diese mit wässrigen Chitosanlösungen behandelt.

Überraschenderweise wurde gefunden, dass der Zusatz von Carotinverbindungen zu Vacciniumextrakten, speziell Extrakten des *Vaccinium myrtillus*, bei oraler Aufnahme zu einer synergistischen Verbesserung des Sehvermögens, speziell des Nachtsehvermögens führt. Die Erfindung schließt die Erkenntnis ein, dass man als Komponente (a) an Stelle der Extrakte auch die wesentlichen Wirkstoffe, bei denen es sich um Anthocyanoside im Allgemeinen und speziell Delphinidin handelt, einsetzen kann. Zur zeitverzögerten Freisetzung werden die Wirkstoffmischungen in speziellen Mikrokapseln eingesetzt.

### Vaccinium-Extrakte

Unter der Gattung Vaccinium, deren Extrakte die Komponente (a) bilden, versteht der Botaniker eine Gruppe von rund 450 Spezies, von denen *Vaccinium myrtillus*, die gemeine Heidel-oder Blaubeere, die wichtigste ist. Weitere bekannte Spezies sind *Vaccinium angustifollium*, *Vaccinium arboreum, Vaccinium arctostaphylos, Vaccinium caespitosum, Vaccinium corymbosum, Vaccinium hirsutum, Vaccinium macrocarpum, Vaccinium ovatum, Vaccinium oxycoccus, Vaccinium stamineum, Vaccinium uliginosum* sowie *Vaccinium vitis idaea*. Als aktive Wirkstoffe enthalten die *Vaccinium*-Extrakte eine Mischung aus mindestens 15 verschiedenen Anthocyanosiden, wie beispielsweise Delphinidin:

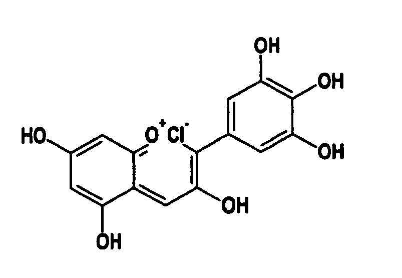

In der Regel enthalten die *Vaccinium*-Extrakte 20 bis 25 Gew.-% Anthocyanoside, 5 bis 10 Gew.-% Tannine sowie in geringen Mengen verschiedene Alkaloide (z.B. Myrtin und Epimyrtin), Phenolsäuren sowie Glycoside mit Quercitrin, Isoquercitrin und Hyperosid.

### Extraktion

Die Herstellung der Extrakte kann in an sich bekannter Weise erfolgen, d.h. beispielsweise durch wässrigen, alkoholischen oder wässrig-alkoholischen Auszug der Pflanzen bzw. Pflanzenteile bzw. der Blätter oder Früchte. Geeignet sind alle herkömmlichen Extraktionsverfahren wie z.B. Mazeration, Remazeration, Digestion, Bewegungsmazeration, Wirbelextraktion, Ultraschallextraktion, Gegenstromextraktion, Perkolation, Reperkolation, Evakolation (Extraktion unter vermindertem Druck), Diakolation oder Festflüssig-Extraktion unter kontinuierlichem Rückfluss. Für den großtechnischen Einsatz vorteilhaft ist die Perkolationsmethode. Als Ausgangsmaterial können frische Pflanzen oder Pflanzenteile eingesetzt werden, üblicherweise wird jedoch von getrockneten Pflanzen und/oder Pflanzenteilen ausgegangen, die vor der Extraktion mechanisch zerkleinert werden können. Hierbei eignen sich alle dem Fachmann bekannten Zerkleinerungsmethoden, als Beispiel sei die Gefriermahlung genannt. Als Lösungsmittel für die Durchführung der Extraktionen können organische Lösungsmittel, Wasser (vorzugsweise heißes Wasser einer Temperatur von über 80 °C und insbesondere von über 95 °C) oder Gemische aus organischen Lösungsmitteln und Wasser, insbesondere niedermolekulare Alkohole mit mehr oder weniger hohen Wassergehalten, verwendet werden. Besonders bevorzugt ist die Extraktion mit Methanol, Ethanol, Pentan, Hexan, Heptan, Aceton, Propylenglykolen, Polyethylenglykolen sowie Ethylacetat sowie Mischungen hieraus sowie deren wässrige Gemische. Die Extraktion erfolgt in der Regel bei 20 bis 100 °C, bevorzugt bei 30 bis 90 °C, insbesondere bei 60 bis 80 °C. In einer bevorzugten Ausführungsform erfolgt die Extraktion unter Inertgasatmosphäre zur Vermeidung der Oxidation der Wirkstoffe des Extraktes. Dies ist insbesondere bei Extraktionen bei Temperaturen über 40 °C von Bedeutung. Die Extraktionszeiten werden vom Fachmann in Abhängigkeit vom Ausgangsmaterial, dem Extraktionsverfahren, der Extraktionstemperatur, vom Verhältnis Lösungsmittel zu Rohstoff u.a. eingestellt. Nach der Extraktion können die erhaltenen Rohextrakte gegebenenfalls weiteren üblichen Schritten, wie beispielsweise Aufreinigung, Konzentration und/oder Entfärbung unterzogen werden. Falls wünschenswert, können die so hergestellten Extrakte beispielsweise einer selektiven Abtrennung einzelner unerwünschter Inhaltsstoffe, unterzogen werden. Die Extraktion kann bis zu jedem beliebigen Extraktionsgrad erfolgen, wird aber gewöhnlich bis zur Erschöpfung durchgeführt. Typische Ausbeuten (= Trockensubstanzmenge des Extraktes bezogen auf eingesetzte Rohstoffmenge) bei der Extraktion getrockneter Blätter liegen im Bereich von 3 bis 15, insbesondere 6 bis 10 Gew.-%. Die vorliegende Erfindung umfasst die Erkenntnis, dass die Extraktionsbedingungen sowie die Ausbeuten der Endextrakte vom Fachmann ja nach gewünschtem Einsatzgebiet gewählt werden können. Diese Extrakte, die in der Regel Aktivsubstanzgehalte (= Feststoffgehalte) im Bereich von 0,5 bis 10 Gew.-% aufweisen, können als solche eingesetzt werden, es ist jedoch ebenfalls möglich, das Lösungsmittel durch Trocknung, insbesondere durch Sprüh- oder Gefriertrocknung vollständig zu entfernen, wobei ein intensiv rot gefärbter Feststoff zurückbleibt. Die Extrakte können auch als Ausgangsstoffe für die Gewinnung der oben genannten reinen Wirkstoffe dienen, sofern diese nicht auf synthetischem Wege einfacher und kostengünstiger hergestellt werden können. Demzufolge kann der Wirkstoffgehalt in den Extrakten 5 bis 100, vorzugsweise 50 bis 95 Gew.-% betragen. Die Extrakte selbst können als wässrige und/oder in organischen Solventien gelöste Zubereitungen sowie als sprüh- bzw. gefriergetrocknete, wasserfreie Feststoffe vorliegen. Als organische Lösungsmittel kommen in diesem Zusammenhang beispielsweise die aliphatischen Alkohole mit 1 bis 6 Kohlenstoffatomen (z.B. Ethanol), Ketone (z.B. Aceton), Halogenkohlenwasserstoffe (z.B. Chloroform oder Methylenchlorid), niedere Ester oder Polyole (z.B. Glycerin oder Glycole) in Frage.

### Carotinverbindungen

Unter Carotinverbindungen, die die Komponente (b) bilden, sind im wesentlichen Carotine und Carotinoide zu verstehen. Carotine stellen eine Gruppe von 11- bis 12fach ungesättigten Triterpenen dar. Von besonderer Bedeutung sind die drei isomeren α-, β- und γ-Carotine, die alle über das gleiche Grundgerüst mit 9 konjugierten Doppelbindungen, 8 Methylverzweigungen (einschließlich möglicher Ringstrukturen) und einer β-Ionon-Ringstruktur an einem Molekülende verfügen und ursprünglich als einheitlicher Naturstoff angesehen worden waren.

Nachstehend sind eine Reihe von Carotinverbindungen abgebildet, die als Komponente (b) in Frage kommen, ohne dass es sich um eine abschließende Aufzählung handelt.

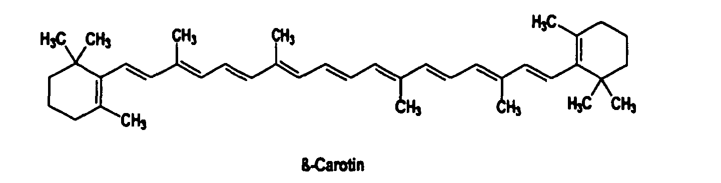

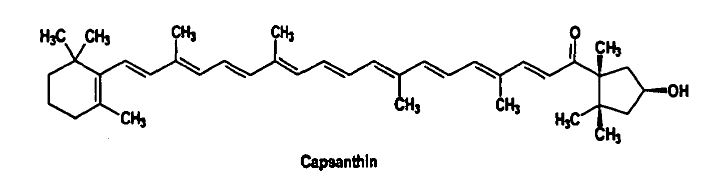

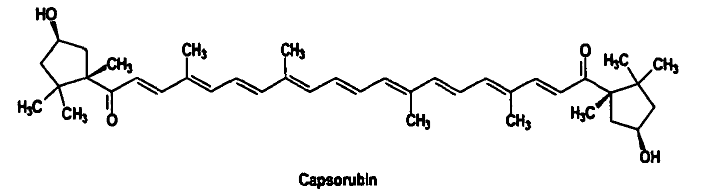

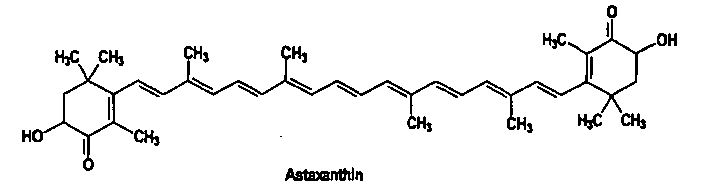

Neben den bereits genannten Isomeren kommen auch das δ-, ε- und ζ-Carotin (Lycopin) in Betracht, wobei freilich das β-Carotin (Provitamin A) wegen seiner hohen Verbreitung von besonderer Bedeutung ist; im Organismus wird es enzymatisch in zwei Moleküle Retinal gespalten. Unter Carotinoiden versteht man sauerstoffhaltige Derivate der Carotine, die auch als Xanthophylle bezeichnet werden, und deren Grundgerüst aus 8 Isopreneinheiten (Tetraterpene) bestehen. Man kann sich die Carotinoide aus zwei C₂₀-Isoprenopiden derart zusammengesetzt denken, dass die beiden mittleren Methylgruppen in 1,6-Stellung zueinander stehen. Typische Beispiele sind das (3R,6'R)-β-ε-Carotin-3,3'-diol (Lutein), (3R,3'S,5'R)-3,3'-Dihydroxy-β,κ-carotin-6-on (Capsanthin), das 9'-cis-6,6'-Diapocarotindisäure-6'-methylester (Bixin), (3S,3'S,5R,5'R)-3,3'-Dihydroxy-κ,κ-carotin-6,6'-dion (Capsorubin) oder das 3S,3'S)-3,3'-Dihydroxy-β,β'-carotin-4,4'-dion (Astaxanthin).

Neben den Carotinen und Carotinoiden sollen unter dem Begriff Carotinverbindungen auch deren Spaltprodukte wie beispielsweise 3,7-Dimethyl-9-(2,6,6-trimethyl-1-cyclohexenyl)-2,4,6,8-nonatetraen-1-ol (Retinol, Vitamin A1) und 3,7-Dimethyl-9-(2,6,6-trimethyl-1-cyclohexenyl)-2,4,6,8-nonatetraenal (Retinal, Vitamin A1-Aldehyd) verstanden werden.
Vorzugsweise kommen als Komponente (b) Lutein oder Astaxanthin vor. Das Einsatzverhältnis zwischen den Komponenten (a) und (b) kann bezogen auf Gewichtsteile 90 : 10 bis 10 : 90, vorzugsweise 75 : 25 bis 25 : 75 und insbesondere 60 : 40 bis 40 : 60 betragen.

### Mikrokapseln

Die erfindungsgemäßen Zubereitungen werden oral aufgenommen. Hierzu hat es sich als nützlich erwiesen, die Zubereitungen zu mikroverkapseln, wobei eine verzögerte Freisetzung entweder durch die Poren der Membran oder durch deren allmähliche Auflösung geschieht. Unter dem Begriff "Mikrokapsel" werden vom Fachmann sphärische Aggregate mit einem Durchmesser im Bereich von etwa 0,1 bis etwa 5 mm verstanden, die mindestens einen festen oder flüssigen Kern enthalten, der von mindestens einer kontinuierlichen Hülle umschlossen ist. Genauer gesagt handelt es sich um mit filmbildenden Polymeren umhüllte feindisperse flüssige oder feste Phasen, bei deren Herstellung sich die Polymere nach Emulgierung und Koazervation oder Grenzflächenpolymerisation auf dem einzuhüllenden Material niederschlagen. Nach einem anderen Verfahren werden geschmolzene Wachse in einer Matrix aufgenommen ("microsponge"), die als Mikropartikel zusätzlich mit filmbildenden Polymeren umhüllt sein können. Die mikroskopisch kleinen Kapseln lassen sich wie Pulver trocknen. Neben einkernigen Mikrokapseln sind auch mehrkernige Aggregate, auch Mikrosphären genannt, bekannt, die zwei oder mehr Kerne im kontinuierlichen Hüllmaterial verteilt enthalten. Ein-oder mehrkernige Mikrokapseln können zudem von einer zusätzlichen zweiten, dritten etc. Hülle umschlossen sein. Die Hülle kann aus natürlichen, halbsynthetischen oder synthetischen Materialien bestehen. Natürlich Hüllmaterialien sind beispielsweise Gummi Arabicum, Agar-Agar, Agarose, Maltodextrine, Alginsäure bzw. ihre Salze, z.B. Natrium- oder Calciumalginat, Fette und Fettsäuren, Cetylalkohol, Collagen, Chitosan, Lecithine, Gelatine, Albumin, Schellack, Polysaccaride, wie Stärke oder Dextran, Polypeptide, Proteinhydrolysate, Sucrose und Wachse. Halbsynthetische Hüllmaterialien sind unter anderem chemisch modifizierte Cellulosen, insbesondere Celluloseester und -ether, z.B. Celluloseacetat, Ethylcellulose, Hydroxypropylcellulose, Hydroxypropylmethylcellulose und Carboxymethylcellulose, sowie Stärkederivate, insbesondere Stärkeether und -ester. Synthetische Hüllmaterialien sind beispielsweise Polymere wie Polyacrylate, Polyamide, Polyvinylalkohol oder Polyvinylpyrrolidon.

Beispiele für Mikrokapseln des Stands der Technik sind folgende Handelsprodukte (in Klammern angegeben ist jeweils das Hüllmaterial) : *Hallcrest Microcapsules* (Gelatine, Gummi Arabicum), *Coletica Thalaspheres* (maritimes Collagen), *Lipotec Millicapseln* (Alginsäure, Agar-Agar), *Induchem Unispheres* (Lactose, mikrokristalline Cellulose, Hydroxypropylmethylcellulose); *Unicerin C30* (Lactose, mikrokristalline Cellulose, Hydroxypropylmethylcellulose), *Kobo Glycospheres* (modifizierte Stärke, Fettsäureester, Phospholipide), *Softspheres* (modifiziertes Agar-Agar), *Kuhs Probiol Nanospheres* (Phospholipide) sowie *Primaspheres* und *Primasponges* (Chitosan, Alginate) und *Primasys* (Phospholipide). Chitosanmikrokapseln und Verfahren zu ihrer Herstellung sind Gegenstand früherer Patenanmeldungen der Patentanmelderin **[**WO 01/01926**,** WO 01/01927**,** WO 01/01928**,** WO 01/01929**].**

Ein weiterer Gegenstand der Erfindung betrifft daher ein Verfahren zur Herstellung von Mikrokapseln mit mittleren Durchmessern im Bereich von 0,1 bis 5 mm, bestehend aus einer Hüllmembran und einer die Wirkstoffe enthaltenden Matrix, bei dem man
(a) aus Gelbildnern, Chitosanen und einer Wirkstoffmischung enthaltend (i) Extrakte der Gattung Vaccinium bzw. Anthocyanoside und (ii) Carotinverbindungen eine Matrix zubereitet und diese mit wässrigen Lösungen anionischer Polymere behandelt
   oder
(b) aus Gelbildnern, anionischen Polymeren und einer Wirkstoffmischung enthaltend (i) Extrakte der Gattung Vaccinium bzw. Anthocyanoside und (ii) Carotinverbindungen eine Matrix zubereitet und diese mit wässrigen Chitosanlösungen behandelt.

### Gelbildner

Im Sinne der Erfindung werden als Gelbildner vorzugsweise solche Stoffe in Betracht gezogen, welche die Eigenschaft zeigen in wässriger Lösung bei Temperaturen oberhalb von 40 °C Gele zu bilden. Typische Beispiele hierfür sind Heteropolysaccharide und Proteine. Als thermogelierende Heteropolysaccharide kommen vorzugsweise Agarosen in Frage, welche in Form des aus Rotalgen zu gewinnenden Agar-Agar auch zusammen mit bis zu 30 Gew.-% nicht-gelbildenden Agaropektinen vorliegen können. Hauptbestandteil der Agarosen sind lineare Polysaccharide aus D-Galaktose und 3,6-Anhydro-L-galaktose, die alternierend β-1,3-und β-1,4-glykosidisch verknüpft sind. Die Heteropolysaccharide besitzen vorzugsweise ein Molekulargewicht im Bereich von 110.000 bis 160.000 und sind sowohl farb- als auch geschmacklos. Als Alternativen kommen Pektine, Xanthane (auch Xanthan Gum) sowie deren Mischungen in Frage. Es sind weiterhin solche Typen bevorzugt, die noch in 1-Gew.-%iger wässriger Lösung Gele bilden, die nicht unterhalb von 80 °C schmelzen und sich bereits oberhalb von 40 °C wieder verfestigen. Aus der Gruppe der thermogelierenden Proteine seien exemplarisch die verschiedenen Gelatine-Typen genannt.

### Chitosane

Chitosane stellen Biopolymere dar und werden zur Gruppe der Hydrokolloide gezählt. Chemisch betrachtet handelt es sich um partiell deacetylierte Chitine unterschiedlichen Molekulargewichtes, die den folgenden - idealisierten - Monomerbaustein enthalten:

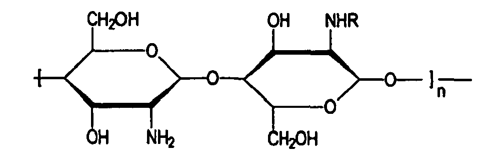

Im Gegensatz zu den meisten Hydrokolloiden, die im Bereich biologischer pH-Werte negativ geladen sind, stellen Chitosane unter diesen Bedingungen kationische Biopolymere dar. Die positiv geladenen Chitosane können mit entgegengesetzt geladenen Oberflächen in Wechselwirkung treten und werden daher in kosmetischen Haar- und Körperpflegemitteln sowie pharmazeutischen Zubereitungen eingesetzt. Zur Herstellung der Chitosane geht man von Chitin, vorzugsweise den Schalenresten von Krustentieren aus, die als billige Rohstoffe in großen Mengen zur Verfügung stehen. Das Chitin wird dabei in einem Verfahren, das erstmals von Hackmann et al. beschrieben worden ist, üblicherweise zunächst durch Zusatz von Basen deproteiniert, durch Zugabe von Mineralsäuren demineralisiert und schließlich durch Zugabe von starken Basen deacetyliert, wobei die Molekulargewichte über ein breites Spektrum verteilt sein können. Vorzugsweise werden solche Typen eingesetzt, wie die ein durchschnittliches Molekulargewicht von 10.000 bis 500.000 bzw. 800.000 bis 1.200.000 Dalton aufweisen und/oder eine Viskosität nach Brookfield (1 Gew.-%ig in Glycolsäure) unterhalb von 5000 mPas, einen Deacetylierungsgrad im Bereich von 80 bis 88 % und einem Aschegehalt von weniger als 0,3 Gew.-% besitzen. Aus Gründen der besseren Wasserlöslichkeit werden die Chitosane in der Regel in Form ihrer Salze, vorzugsweise als Glycolate eingesetzt.

### Ölphase

Die Matrix kann vor der Bildung der Membran optional in einer Ölphase dispergiert werden. Als Öle kommen für diesen Zweck beispielsweise Guerbetalkohole auf Basis von Fettallcoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, I-sostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von Hydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di /Triglycerid-mischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, Guerbetcarbonate, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, Ringöffnungsprodukte von epoxidierten Fettsäureestern mit Polyolen, Siliconöle und/oder aliphatische bzw. naphthenische Kohlenwasserstoffe, wie z.B. wie Squalan, Squalen oder Dialkylcyclohexane in Betracht.

### Anionpolymere

Die anionischen Polymere haben die Aufgabe, mit den Chitosanen Membranen zu bilden. Für diesen Zweck eignen sich vorzugsweise Salze der Alginsäure. Bei der Alginsäure handelt es sich um ein Gemisch carboxylgruppenhaltiger Polysaccharide mit folgendem idealisierten Monomerbaustein:

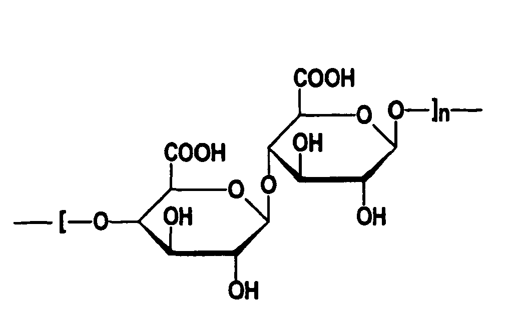

Das durchschnittliche Molekulargewicht der Alginsäuren bzw. der Alginate liegt im Bereich von 150.000 bis 250.000. Dabei sind als Salze der Alginsäure sowohl deren vollständige als auch deren partiellen Neutralisationsprodukte zu verstehen, insbesondere die Alkalisalze und hierunter vorzugsweise das Natriumalginat ("Algin") sowie die Ammonium- und Erdalkalisalze. besonders bevorzugt sind Mischalginate, wie z.B. Natrium/Magnesium- oder Natrium/Calciumalginate. In einer alternativen Ausführungsform der Erfindung kommen für diesen Zweck jedoch auch anionische Chitosanderivate, wie z.B. Carboxylierungs- und vor allem Succinylierungsprodukte in Frage. Alternativ kommen auch Poly(meth)acrylate mit durchschnittlichen Molekulargewichten im Bereich von 5.000 bis 50.000 Dalton sowie die verschiedenen Carboxymethylcellulosen in Frage. Anstelle der anionischen Polymeren können für die Ausbildung der Hüllmembran auch anionische Tenside oder niedermolekulare anorganische Salze, wie beispielsweise Pyrophosphate eingesetzt werden.

### Herstellverfahren Mikrokapseln

Zur Herstellung der Mikrokapseln stellt man üblicherweise eine 1 bis 10, vorzugsweise 2 bis 5 Gew.-%ige wässrige Lösung des Gelbildners, vorzugsweise des Agar-Agars her und erhitzt diese unter Rückfluss. In der Siedehitze, vorzugsweise bei 80 bis 100°C, wird eine zweite wässrige Lösung zugegeben, welche das Chitosan in Mengen von 0,1 bis 2, vorzugsweise 0,25 bis 0,5 Gew.-% und den Wirkstoff (Komponenten a und b) in Mengen von 0,1 bis 25 und insbesondere 0,25 bis 10 Gew.-% enthält; diese Mischung wird als Matrix bezeichnet. Die Beladung der Mikrokapseln mit Wirkstoffen kann daher ebenfalls 0,1 bis 25 Gew.-% bezogen auf das Kapselgewicht betragen. Falls gewünscht, können zu diesem Zeitpunkt zur Viskositätseinstellung auch wasserunlösliche Bestandteile, beispielsweise anorganische Pigmente zugegeben werden, wobei man diese in der Regel in Form von wässrigen oder wässrig/alkoholischen Dispersionen zusetzt. Zur Emulgierung bzw. Dispergierung der Wirkstoffe kann es ferner von Nutzen sein, der Matrix Emulgatoren und/oder Lösungsvermittler hinzu zu geben. Nach der Herstellung der Matrix aus Gelbildner, Chitosan und Wirkstoffmischung (Komponenten a und b) kann die Matrix optional in einer Ölphase unter starker Scherung sehr fein dispergiert werden, um bei der nachfolgenden Verkapselung möglichst kleine Teilchen herzustellen. Dabei hat es sich als besonders vorteilhaft erwiesen, die Matrix auf Temperaturen im Bereich von 40 bis 60 °C zu erwärmen, während man die Ölphase auf 10 bis 20 °C kühlt. Im letzten, nun wieder obligatorischen Schritt erfolgt dann die eigentliche Verkapselung, d.h. die Ausbildung der Hüllmembran durch Inkontaktbringen des Chitosans in der Matrix mit den anionischen Polymeren. Hierzu empfiehlt es sich, die gegebenenfalls in der Ölphase dispergierte Matrix bei einer Temperatur im Bereich von 40 bis 100, vorzugsweise 50 bis 60 °C mit einer wässrigen, etwa 1 bis 50 und vorzugsweise 10 bis 15 Gew.-%ige wässrigen Lösung des Anionpolymers zu behandeln und dabei - falls erforderlich - gleichzeitig oder nachträglich die Ölphase zu entfernen. Die dabei resultierenden wässrigen Zubereitungen weisen in der Regel einen Mikrokapselgehalt im Bereich von 1 bis 10 Gew.-% auf. In manchen Fällen kann es dabei von Vorteil sein, wenn die Lösung der Polymeren weitere Inhaltsstoffe, beispielsweise Emulgatoren oder Konservierungsmittel enthält. Nach Filtration werden Mikrokapseln erhalten, welche im Mittel einen Durchmesser im Bereich von vorzugsweise etwa 1 mm aufweisen. Es empfiehlt sich, die Kapseln zu sieben, um eine möglichst gleichmäßige Größenverteilung sicherzustellen. Die so erhaltenen Mikrokapseln können im herstellungsbedingten Rahmen eine beliebige Form aufweisen, sie sind jedoch bevorzugt näherungsweise kugelförmig. Alternativ kann man die Anionpolymere auch zur Herstellung der Matrix einsetzen und die Verkapselung mit den Chitosanen durchführen.

### Gewerbliche Anwendbarkeit

Ein letzter Gegenstand der Erfindung betrifft die Verwendung erfindungsgemäßen Mikrokapseln zur Herstellung eines Medikamentes zur Stärkung des menschlichen Sehvermögens.

### Beispiele

### Beispiel 1

In einem 500-ml-Dreihalskolben mit Rührer und Rückflusskühler wurden in der Siedehitze 3 g Agar-Agar in 200 ml Wasser gelöst. Anschließend wurde die Mischung innerhalb von etwa 30 min unter starkem Rühren zunächst mit einer homogenen Dispersionen von 10 g Glycerin und 2 g Talk in ad 100 g Wasser und dann mit einer Zubereitung von 25 g Chitosan (Hydagen® DCMF, 1 Gew.-%ig in Glycolsäure, Cognis, Düsseldorf/FRG), 8 g eines wässrigen, 10 Gew.-%igen Extraktes von *Vaccinium myrtillus*, 0,1 g Lutein, 0,5 g Phenonip® (Konservierungsmittelmischung enthaltend Phenoxyethanol und Parabene) und 0,5 g Polysorbat-20 (Tween® 20, ICI) in ad 100 g Wasser versetzt. Die erhaltene Matrix wurde filtriert, auf 60 °C erwärmt und in eine 0,5 Gew.-%ige Natriumalginatlösung getropft. Nach dem Sieben wurde eine wässrige Zubereitung erhalten, die 8 Gew.-% Mikrokapseln mit einem mittleren Durchmesser von 1 mm enthielt.

### Beispiel 2

In einem 500-ml-Dreihalskolben mit Rührer und Rückflusskühler wurden in der Siedehitze 3 g Agar-Agar in 200 ml Wasser gelöst. Anschließend wurde die Mischung innerhalb von etwa 30 min unter starkem Rühren zunächst mit einer homogenen Dispersionen von 10 g Glycerin und 2 g Talk in ad 100 g Wasser und dann mit einer Zubereitung von 25 g Chitosan (Hydagen® DCMF, 1 Gew.-%ig in Glycolsäure, Cognis, Düsseldorf/FRG), 8 g eines wässrigen, 10 Gew.-%igen Extraktes von *Vaccinium myrtillus*, 0,1 g Astaxanthin, 0,5 g Phenonip® (Konservierungsmittelmischung enthaltend Phenoxyethanol und Parabene) und 0,5 g Polysorbat-20 (Tween® 20, ICI) in ad 100 g Wasser versetzt. Die erhaltene Matrix wurde filtriert, auf 50 °C temperiert und unter starkem Rühren im 2,5fachen Volumen Paraffinöl, das zuvor auf 15 °C gekühlt worden war, dispergiert. Die Dispersion wurde anschließend mit einer wässrigen Lösung enthaltend 1 Gew.-% Natriumlaurylsulfat und 0,5 Gew.-% Natriumalginat und dann mehrfach mit einer 0,5 Gew.-%igen wässrigen Phenoniplösung gewaschen, wobei die Ölphase entfernt wurde. Nach dem Sieben wurde eine wässrige Zubereitung erhalten, die 8 Gew.-% Mikrokapseln mit einem mittleren Durchmesser von 1 mm enthielt.

### Beispiel 3

In einem 500-ml-Dreihalskolben mit Rührer und Rückflusskühler wurden in der Siedehitze 3 g Agar-Agar in 200 ml Wasser gelöst. Anschließend wurde die Mischung innerhalb von etwa 30 min unter starkem Rühren zunächst mit einer homogenen Dispersionen von 10 g Glycerin und 2 g Talk in ad 100 g Wasser und dann mit einer Zubereitung von 25 g Chitosan (Hydagen® DCMF, 1 Gew.-%ig in Glycolsäure, Cognis, Düsseldorf/FRG), 8 g eines wässrigen, 10 Gew.-%igen Extraktes von *Vaccinium myrtillus*, 0,5 g Beta-Carotin, 0,5 g Phenonip® (Konservierungsmittelmischung enthaltend Phenoxyethanol und Parabene) und 0,5 g Polysorbat-20 (Tween® 20, ICI) in ad 100 g Wasser versetzt. Die erhaltene Matrix wurde filtriert, auf 60 °C erwärmt und in eine 15 Gew.-%ige Lösung von Sodium Laureth Sulfate getropft. Nach dem Sieben wurde eine wässrige Zubereitung erhalten, die 9 Gew.-% Mikrokapseln mit einem mittleren Durchmesser von 1 mm enthielt.

### Beispiel 4

In einem 500-ml-Dreihalskolben mit Rührer und Rückflusskühler wurden in der Siedehitze 3 g Agar-Agar in 200 ml Wasser gelöst. Anschließend wurde die Mischung innerhalb von etwa 30 min unter starkem Rühren zunächst mit einer homogenen Dispersionen von 10 g Glycerin und 2 g Talk in ad 100 g Wasser und dann mit einer Zubereitung von 25 g Chitosan (Hydagen® DCMF, 1 Gew.-%ig in Glycolsäure, Cognis, Düsseldorf/FRG), 8 g eines wässrigen, 10 Gew.-%igen Extraktes von *Vaccinium myrtillus*, 0,3 g Retinol, 0,5 g Phenonip® (Konservierungsmittelmischung enthaltend Phenoxyethanol und Parabene) und 0,5 g Polysorbat-20 (Tween® 20, ICI) in ad 100 g Wasser versetzt. Die erhaltene Matrix wurde filtriert, auf 60 °C erwärmt und in eine 15 Gew.-%ige Lösung von Natriumpyrophosphat getropft. Nach dem Sieben wurde eine wässrige Zubereitung erhalten, die 8 Gew.-% Mikrokapseln mit einem mittleren Durchmesser von 1 mm enthielt.

### Beispiel 5

In einem 500-ml-Dreihalskolben mit Rührer und Rückflusskühler wurden in der Siedehitze 3 g Agar-Agar in 200 ml Wasser gelöst. Anschließend wurde die Mischung innerhalb von etwa 30 min unter starkem Rühren zunächst mit einer homogenen Dispersionen von 10 g Glycerin und 2 g Talk in ad 100 g Wasser und dann mit einer Zubereitung von 25 g Chitosan (Hydagen® DCMF, 1 Gew.-%ig in Glycolsäure, Cognis, Düsseldorf/FRG), 8 g eines wässrigen, 10 Gew.-%igen Extraktes von *Vaccinium myrtillus*, 0,2 g Retinal, 0,5 g Phenonip® (Konservierungsmittelmischung enthaltend Phenoxyethanol und Parabene) und 0,5 g Polysorbat-20 (Tween® 20, ICI) in ad 100 g Wasser versetzt. Die so erhaltene Matrix wurde filtriert, auf 50 °C temperiert und unter starkem Rühren im 2,5fachen Volumen Paraffinöl, das zuvor auf 15 °C gekühlt worden war, dispergiert. Die Dispersion wurde anschließend mit einer 15 Gew.-%igen Natriumpyrophosphatlösung und dann mehrfach mit einer 0,5 Gew.-%igen wässrigen Phenoniplösung gewaschen, wobei die Ölphase entfernt wurde. Nach dem Sieben wurde eine wässrige Zubereitung erhalten, die 10 Gew.-% Mikrokapseln mit einem mittleren Durchmesser von 1 mm enthielt.

### Beispiel 6

In einem 500-ml-Dreihalskolben mit Rührer und Rückflusskühler wurden in der Siedehitze 3 g Gelatine in 200 ml Wasser gelöst. Anschließend wurde die Mischung innerhalb von etwa 30 min unter starkem Rühren zunächst mit einer homogenen Dispersionen von 10 g Glycerin und 2 g Talk in ad 100 g Wasser und dann mit einer Zubereitung von 25 g Chitosan (Hydagen® DCMF, 1 Gew.-%ig in Glycolsäure, Henkel KGaA, Düsseldorf/FRG), 8 g eines wässrigen, 10 Gew.-%igen Extraktes von *Vaccinium myrtillus*, 0,1 g Lutein, 0,1 g Astaxanthin, 0,5 g Phenonip® in ad 100 g Wasser versetzt. Die erhaltene Matrix wurde filtriert, auf 60 °C erwärmt und in eine 0,5 Gew.-%ige Lösung von Hydagen® SCD (succinyliertes Chitosan, Cognis) getropft. Nach dem Sieben wurde eine wässrige Zubereitung erhalten, die 8 Gew.-% Mikrokapseln mit einem mittleren Durchmesser von 1 mm enthielt.

### Beispiel 7

In einem 500-ml-Dreihalskolben mit Rührer und Rückflusskühler wurden in der Siedehitze 3 g Agar-Agar in 200 ml Wasser gelöst. Anschließend wurde die Mischung innerhalb von etwa 30 min unter starkem Rühren zunächst mit einer homogenen Dispersionen von 10 g Glycerin und 2 g Talk in ad 100 g Wasser und dann mit einer Zubereitung von 25 g Chitosan (Hydagen® DCMF, 1 Gew.-%ig in Glycolsäure, Cognis, Düsseldorf/FRG), 8 g eines wässrigen, 10 Gew.-%igen Extraktes von *Vaccinium myrtillus*, 0,1 g Lutein, 0,5 g Beta-Carotin, 0,5 g Phenonip® (Konservierungsmittelmischung enthaltend Phenoxyethanol und Parabene) und 0,5 g Polysorbat-20 (Tween® 20, ICI) in ad 100 g Wasser versetzt. Die erhaltene Matrix wurde filtriert, auf 60 °C erwärmt und in eine 0,5 Gew.-%ige Natriumalginatlösung getropft. Zum Erhalt von Mikrokapseln gleichen Durchmessers wurden die Zubereitungen anschließend gesiebt.

## Patentansprüche

1. Mikrokapseln mit mittleren Durchmessern im Bereich von 0,1 bis 5 mm, bestehend aus einer Hüllmembran und einer die Wirkstoffe enthaltenden Matrix, **dadurch** erhältlich, dass man
(a) aus Gelbildnern, Chitosanen und einer Wirkstoffmischung enthaltend (i) Extrakte der Gattung Vaccinium bzw. Anthocyanoside und (ii) Carotinverbindungen eine Matrix zubereitet, und diese mit wässrigen Lösungen anionischer Polymere behandelt;
oder
(b) aus Gelbildnern, anionischen Polymeren und einer Wirkstoffmischung enthaltend (i) Extrakte der Gattung Vaccinium bzw. Anthocyanoside und (ii) Carotinverbindungen eine Matrix zubereitet und diese mit wässrigen Chitosanlösungen behandelt.

2. Verfahren zur Herstellung von Mikrokapseln mit mittleren Durchmessern im Bereich von 0,1 bis 5 mm, bestehend aus einer Hüllmembran und einer die Wirkstoffe enthaltenden Matrix, bei dem man
(a) aus Gelbildnern, Chitosanen und einer Wirkstoffmischung enthaltend (i) Extrakte der Gattung Vaccinium bzw. Anthocyanoside und (ii) Carotinverbindungen eine Matrix zubereitet und diese mit wässrigen Lösungen anionischer Polymere behandelt
oder
(b) aus Gelbildnern, anionischen Polymeren und einer Wirkstoffmischung enthaltend (i) Extrakte der Gattung Vaccinium bzw. Anthocyanoside und (ii) Carotinverbindungen eine Matrix zubereitet und diese mit wässrigen Chitosanlösungen behandelt.

3. Verfahren nach Anspruch 2, dass man als Wirkstoffkomponente (i) Delphinidin einsetzt.

4. Verfahren nach den Ansprüchen 2 und/oder 3, **dadurch gekennzeichnet, dass** man als Wirkstoffkomponente (ii) Carotinverbindungen einsetzt, die ausgewählt sind aus der Gruppe, die gebildet wird von den isomeren α-, β-, γ-, δ-und ε-Carotinen, Lycopin (ζ-Carotin), Lutein, Capsanthin, Capsorubin, Bixin, Astaxanthin, Retinol und Retinal sowie deren Gemischen.

5. Verfahren nach mindestens einem der Ansprüche 2 bis 4, **dadurch gekennzeichnet, dass** man die Komponenten (i) und (ii) im Gewichtsverhältnis 10:90 bis 90:10 einsetzt.

6. Verfahren nach mindestens einem der Ansprüche 2 bis 5, **dadurch gekennzeichnet, dass** man als Gelbildner Heteropolysaccharide oder Proteine einsetzt.

7. Verfahren nach mindestens einem der Ansprüche 2 bis 6, **dadurch gekennzeichnet, dass** man als Heteropolysaccharide Agarosen, Agar-Agar, Pektine, Xanthane sowie deren Gemische einsetzt.

8. Verfahren nach mindestens einem der Ansprüche 2 bis 7, **dadurch gekennzeichnet, dass** man als Proteine Gelatine einsetzt.

9. Verfahren nach mindestens einem der Ansprüche 2 bis 8, **dadurch gekennzeichnet, dass** man Chitosane einsetzt, die ein mittleres Molekulargewicht im Bereich von 10.000 bis 500.000 bzw. 800.000 bis 1.200.000 Dalton aufweisen.

10. Verfahren nach mindestens einem der Ansprüche 2 bis 9, **dadurch gekennzeichnet, dass** man anionische Polymere einsetzt, die ausgewählt sind aus der Gruppe, die gebildet wird von Salzen der Alginsäure, anionischen Chitosanderivaten, Poly(meth)acrylaten und Carboxymethylcellulosen.

11. Verwendung von Mikrokapseln nach Anspruch 1 zur Herstellung eines Medikamentes zur Stärkung des menschlichen Sehvermögens.

## Claims

1. Microcapsules with mean diameters of 0.1 to 5 mm consisting of a membrane and a matrix containing the active components, **characterized in that** they are obtainable by
(a) preparing a matrix from gel formers, chitosans and an active component mixture containing (i) extracts of the genus Vaccinium or anthocyanosides and (ii) carotene compounds and treating the matrix with aqueous solutions of anionic polymers;
or
(b) preparing a matrix from gel formers, anionic polymers and an active component mixture containing (i) extracts of the genus Vaccinium or anthocyanosides and (ii) carotene compounds and treating the matrix with aqueous chitosan solutions.

2. Process for the production of microcapsules with mean diameters of 0.1 to 5 mm consisting of a membrane and a matrix containing the active components, **characterized in that** it comprises the steps of
(a) preparing a matrix from gel formers, chitosans and an active component mixture containing (i) extracts of the genus Vaccinium or anthocyanosides and (ii) carotene compounds and treating the matrix with aqueous solutions of anionic polymers;
or
(b) preparing a matrix from gel formers, anionic polymers and an active component mixture containing (i) extracts of the genus Vaccinium or anthocyanosides and (ii) carotene compounds and treating the matrix with aqueous chitosan solutions.

3. Process as claimed in claim 2, **characterized in that** delphinidin is used as active component (i).

4. Process as claimed in claims 2 and/or 3, **characterized in that** carotene compounds selected from the group consisting of the isomeric α-, β-, γ-, δ- and ε-carotenes, lycopene (ζ-carotene), lutein, capsanthin, capsorubin, bixin, astaxanthin, retinol and retinal and mixtures thereof are used as active component (ii).

5. Process as claimed in at least one of claims 2 to 4, **characterized in that** components (i) and (ii) are used in a ratio by weight of 10:90 to 90:10.

6. Process as claimed in at least one of claims 2 to 5, **characterized in that** heteropolysaccharides or proteins are used as the gel formers.

7. Process as claimed in at least one of claims 2 to 6, **characterized in that** agaroses, agar agar, pectins, xanthans and mixtures thereof are used as the heteropolysaccharides.

8. Process as claimed in at least one of claims 2 to 7, **characterized in that** gelatine is used as the protein.

9. Process as claimed in at least one of claims 2 to 8, **characterized in that** chitosans with an average molecular weight of 10,000 to 500,000 or 800,000 to 1,200,000 dalton are used.

10. Process as claimed in at least one of claims 2 to 9, **characterized in that** anionic polymers selected from the group consisting of salts of alginic acid, anionic chitosan derivatives, poly(meth)acrylates and carboxymethyl celluloses are used.

11. Use of the microcapsules claimed in claim 1 for the production of a medicament for improving human sight.

## Revendications

1. Microcapsules présentant un diamètre moyen compris dans la plage allant de 0,1 à 5 mm, constituées d'une membrane enveloppe et d'une matrice contenant les principes actifs, pouvant être obtenues par
(a) préparation d'une matrice à partir de gélifiants, de chitosanes et d'un mélange de principes actifs comprenant (i) des extraits du genre Vaccinium ou des anthocyanosides et (ii) des composés de carotène, et traitement de celle-ci avec des solutions aqueuses de polymères anioniques ;
ou
(b) préparation d'une matrice à partir de gélifiants, de polymères anioniques et d'un mélange de principes actifs comprenant (i) des extraits du genre Vaccinium ou des anthocyanosides et (ii) des composés de carotène, et traitement de celle-ci avec des solutions aqueuses de chitosanes.

2. Procédé de préparation de microcapsules présentant un diamètre moyen compris dans la plage allant de 0,1 à 5 mm, constituées d'une membrane enveloppe et d'une matrice contenant les principes actifs, selon lequel
(a) on prépare une matrice à partir de gélifiants, de chitosanes et d'un mélange de principes actifs comprenant (i) des extraits du genre Vaccinium ou des anthocyanosides et (ii) des composés de carotène, et on traite celle-ci avec des solutions aqueuses de polymères anioniques ;
ou
(b) on prépare une matrice à partir de gélifiants, de polymères anioniques et d'un mélange de principes actifs comprenant (i) des extraits du genre Vaccinium ou des anthocyanosides et (ii) des composés de carotène, et on traite celle-ci avec des solutions aqueuses de chitosanes.

3. Procédé selon la revendication 2,
**caractérisé en ce qu'**
comme composé actif (i) on utilise de la delphinidine.

4. Procédé selon les revendications 2 et/ou 3,
**caractérisé en ce qu'**
comme composé actif (ii) on utilise des composés de carotène choisis dans le groupe constitué par les isomères α-, β-, γ-, δ-, et ε-carotènes, le lycopène (ζ-carotène), la lutéine, la capsanthine, la capsorubine, la bixine, l'astaxanthine, le rétinol et le rétinal ainsi que leurs mélanges.

5. Procédé selon l'une au moins des revendications 2 à 4,
**caractérisé en ce qu'**
on utilise les composants (i) et (ii) dans un rapport pondéral allant de 10 : 90 à 90 : 10.

6. Procédé selon l'une au moins des revendications 2 à 5,
**caractérisé en ce qu'**
comme gélifiants on utilise des hétéropolysaccharides ou des protéines.

7. Procédé selon l'une au moins des revendications 2 à 6,
**caractérisé en ce qu'**
comme hétéropolysaccharides on utilise des agaroses, de l'agar-agar, des pectines, des xanthanes ainsi que leurs mélanges.

8. Procédé selon l'une au moins des revendications 2 à 7,
**caractérisé en ce qu'**
comme protéines on utilise de la gélatine.

9. Procédé selon l'une au moins des revendications 2 à 8,
**caractérisé en ce qu'**
on utilise des chitosanes présentant un poids moléculaire moyen compris dans la plage allant de 10 000 à 500 000 ou de 800 000 à 1 200 000 dalton.

10. Procédé selon l'une au moins des revendications 2 à 9,
**caractérisé en ce qu'**
on utilise des polymères anioniques choisis dans le groupe constitué par les sels de l'acide alginique, les dérivés anioniques de chitosane, les poly(méth)acrylates et les carboxyméthylcelluloses.

11. Utilisation des microcapsules selon la revendication 1 pour la préparation d'un médicament visant à augmenter la vision chez l'homme.
